# EUROPÄISCHE PATENTSCHRIFT

(11) **EP 0 406 586 B1**
(45) Veröffentlichungstag und Bekanntmachung des Hinweises auf die Patenterteilung: **20.10.1993**
(21) Anmeldenummer: 90111042.9
(22) Anmeldetag: 12.06.1990
(51) Int. Cl.: A61M 19/00

(54) **Anästhesiebesteck**
Set of instruments for anaesthesia
Trousse pour l'anesthésie

(30) Priorität: 07.07.1989 DE 3922406
(43) Veröffentlichungstag der Anmeldung: 09.01.1991
(73) Patentinhaber: B. Braun Melsungen AG, 34209 Melsungen (DE)
(72) Erfinder: Mononen, Pekka, Dr., . (FI); Plantiko, Peter, Dr., D-3000 Hannover 91 (DE); Haindl, Hans-Günter, Dr., D-3508 Melsungen (DE)
(74) Vertreter: Selting, Günther, Dipl.-Ing.

(56) Entgegenhaltungen:
- WO-A-89/00436
- DE-A- 3 327 585
- DE-A- 3 710 262
- DE-U- 8 811 408
- JP-U- 6 341 007
- Clinical Anesthesia, Vol. 7/Nr. 6,Juni 1983,Kazuo Hanaoka

## Beschreibung

Die Erfindung bezieht sich auf ein Anästhesiebesteck, nach dem Oberbegriff des Patentanspruches 1.

Bei Operationen der unteren Extremität und der Beckenorgane stehen zur Regionalanästhesie im wesentlichen die Spinalanästhesie und die Epiduralanästhesie zur Verfügung. Vorteile der Spinalanästhesie sind schneller Wirkungseintritt, kaum Versager und meist vollständige Analgesie. Die Nachteile bestehen darin, daß sie nur bis etwa Hüfthöhe wirksam ist, daß die Wirkdauer bei einmaliger Injektion (single shot) begrenzt ist, andererseits das Einbringen eines Katheters in den Spinalraum Infektionen hervorrufen kann. Zur Durchführung der Spinalanästhesie dient ein Besteck gemäß DE-U- 88 11 408. Dabei ist die Epiduralkanüle eine als Crawford-Kanüle bekannte Kanüle, deren vordere gerade Spitze kurz abgeschrägt und angeschliffen ist. Die Schliffränder sind zur Verringerung des Risikos der Dura-Perforation gerundet. Durch die koaxiale endständige Öffnung der Epiduralkanüle wird die dünnere Spinalkanüle zur Perforation der Dura vorgeschoben und es wird über einen Führungsdraht der Katheter durch die Epiduralkanüle hindurch in den Spinalraum eingeschoben. Durch den Katheter, an dessen extrakorporalem Ende ein konnektor befestigt ist, kann z.B. mittels einer Spritze, Analgetikum nachinjiziert werden.

Bei der Epiduralanästhesie wird durch eine Epiduralkanüle ein Katheter in den Epiduralraum eingebracht. Dies ist unkritischer als bei der Spinalanästhesie. Beliebige Analgesiedauer durch Nachspritzen und postoperative Analgesie sind erreichbar. Bei Verwendung geeigneter Analgetika kann die Analgesie bei wiederhergestellter motorischer Funktion aufrechterhalten werden. Obwohl die Wirkung später als bei der Spinalanästhesie eintritt, hat sie gegenüber dieser den Vorteil, daß sie bis zur Brusthöhe effektiv ist. Durch Kombination der Spinalanästhesie und der Epiduralanästhesie läßt sich daher bei Summierung der spezifischen Vorzüge eine Ausweitung des Anästhesiebereiches erzielen. Die bekannten Bestecks für die Epiduralanästhesie lassen jedoch eine Kombination nicht gefahrlos zu. Dies ist darauf zurückzuführen, daß die Dura von der durch die Epiduralkanüle hindurchgeschobenen Spinalkanüle zum Einspritzen von Analgetikum perforiert worden ist und daß ein ebenfalls durch die Epiduralkanüle hindurchgeschobener Epiduralkatheter genau an der Stelle auf die Dura trifft, an der diese von der Spinalkanüle vorperforiert worden ist. Dies hat zur Folge, daß der Epiduralkatheter das für den Epiduralraum bestimmte Analgetikum in den Spinalraum einführt, wodurch nicht nur erhöhtes Anästhesierisiko für den Patienten entsteht, sondern der durch die Epiduralanästhesie schmerzfrei zu machende Körperbereich von der Analgesie nicht erfaßt wird.

Bei einem bekannten Besteck (WO89/00436) zur kombinierten Epidural- und Spinalanästhesie wird eine Mehrlumennadel verwendet, bei der das eine, größere Lumen zum Durchlaß eines Epiduralkatheters bestimmt ist und das andere, kleinere Lumen dem Durclaß einer Spinalnadel dient. Der Körper der Mehrlumennadel endet in einer schargen, axialgerichteten Einstechspitze, die dem größeren Lumen zugeordnet ist, das in einer seitwärts gerichteten Öffnung nach Art einer Tuohynadel endet. Die Anordnung von zwei Lumina bedingt einen großen äußeren Durchmesser der Mehrlumennadel und damit ein großes Einstechloch, das das Trauma für den Patienten vergrößert. Außerdem besteht die Gefahr des Durchstechens der Dura mit der Spitze des Epiduralkatheterteiles der Mehrlumennadel neben der Einstichstelle der Spinalkanüle. Diese Gefahr beruht im wesentlichen darauf, daß das Punktionsverhalten der dicken Mehrlumennadel anders ist, als der Anästhesist mit Standardnadeln beim Auffinden des Epiduralraumes gewohnt ist. Der Durchtrittswiderstand bei der Punktion gibt dem Anästhesisten die zur Erkennung der Position der Nadelspitze nötigen Informationen; ist das Gefühl für den Durch- trittswiderstand anders als üblich, so sind die Informationen bezüglich der Nadlvordringung nicht mehr zuver- lässig. Duraverletzungen können nicht nur Kopfschmerz erzeugen, sondern die Öffnung in der Dura kann Medikament in den Spinalraum einlassen, das durch den Epiduralkathether ausschließlich in den Epiduralraum eingeführt werden darf. Insgesamt wird bei Anwendung dieses bekannten Bestecks das Risiko der kombinierten Epidural- und Spinalanästhesie größer.

Ein weiteres Besteck zur kombinierten Epidural- und Spinalanästhesie ist gezeigt in der japanischen Zeitschrift "Clinical Anesthesia", Vol. 7/ Nr.6, Juni 1983, Klinischer Bericht von Kazuo Hanaoka.Bei der Epiduralkanüle handelt es sich um eine Tuohy-Nadel, bei der eine erste Wand unter Bildung einer Abknickung gegen die gegenüberliegende zweite Wand gezogen ist, damit eine seitliche Öffnung entsteht, die etwa in der Ebene der zweiten Wand verläuft. In dem schrägen Abschnitt der ersten Wand der Epiduralkanüle ist außerhalb der Längsmittelachse der Epiduralkanüle ein Loch zum Durchlaß der Spinalkanüle vorgesehen. Die exzentrische Anordnung des Loches macht es erforderlich, daß die Spinalkanüle von ihrem mit einem distalen Kanülenansatz der Epiduralkanüle zusammengestecken Ansatz schräg zu dem Loch in der Epiduralkanülenspitze vorgeschoben werden muß. Es bedarf beträchtlicher Geschicklichkeit des Anwenders, um mit der Spitze der Spinalkanüle das Loch in der Wand der Epiduralkanüle zu treffen. Wenn hierzu mehrere Versuche nötig sind, stößt die sehr feine Spitze der dünnen Spinalkanüle gegen die Innenfläche der Abschrägung der Wand der Epiduralkanüle und wird beschädigt - die Folge ist eine Erschwernis bei der Perforation der Dura mit ungünstigen Auswirkungen auf den Patienten.

Außerdem ist durch JP-U-63-41007 ein Anästhesiebesteck mit den Merkmalen des Oberbegriffes des Patentanspruches 1 bekannt. Auch dieses Anästhesiebesteck zeigt bei der Anwendung Mängel, weil die Perforation der Dura mit der Spinalkanüle nicht präzise durchführbar ist und weil die Gefahr besteht, daß der Epiduralkatheter genau an der Stelle auf die Dura trifft, an der diese von der Spinalkanüle vorperforiert wurde - mit der Folge, daß für den Epiduralraum bestimmtes Analgeticum in den Spinalraum gelangt. Diese Nachteile ergeben sich einmal dadurch, daß der Außendurchmesser der Spinalkanüle sich herstellungsmäßig nicht so anpassen läßt, daß der Vorschub der Spinalkanüle zentrisch geführt erfolgt, d.h. die Spinalkanüle hat in der Epiduralkanüle leichtes Spiel und es besteht die Gefahr, daß sie nicht zentrisch durch das Loch in der Krümmung der Spitze der Epiduralkanüle hindurchtritt, sondern sich verklemmt. Zum anderen ist das Loch in der Spitze der Epiduralkanüle mindestens genauso groß wie der Außendurchmesser des Epiduralkatheters und die gewünschte Umlenkung der Katheterspitze an der Krümmung der Epiduralkanüle ist fraglich. Der Epiduralkatheter kann durch das Loch und die Perforationsstelle der Dura geradenwegs in den Spinalraum gelangen und in diesen für den Epiduralraum bestimmtes Analgeticum einbringen, ohne daß es der Anwender merkt.

Der Erfindung liegt die Aufgabe zugrunde, ein Anästhesiebesteck der erwähnten Art so auszubilden, daß es eine einfache und risikolose Anwendung für die kombinierte Spinal- und Epiduralanästhesie ermöglicht.

Diese Aufgabe wird erfindungsgemäß durch die Merkmale des Patentanspruches 1 gelöst,.

Mit dem erfindungsgemäßen Anästhesiebesteck wird zunächst die Epiduralkanüle, deren Lumen zur Vermeidung von Ausstanzungen durch einen Mandrin ausgefüllt ist, durch Muskelgewebe hindurch bis zur Außenwand der Dura in den Epiduralraum vorgeschoben. Danach wird der Mandrin entfernt und es wird in die Epiduralkanüle die dünnere und längere Spinalkanüle mit Mandrin eingeführt. Diese tritt aus dem koaxialen Loch in der Krümmung der Epiduralkanüle aus und perforiert dort die Dura. Nach Herausziehen des Mandrins aus der Spinalkanüle wird Analgetikum in den Spinalkanal eingespritzt. Die Spinalkanüle wird aus der Epiduralkanüle herausgezogen und ein Epiduralkatheter wird durch die Epiduralkanüle vorgeschoben. Der Epiduralkatheter, der nicht durch das koaxiale Loch in der Epiduralkanüle hindurchpaßt, wird der Krümmung der Spitze der Epiduralkanüle folgend seitlich abgelenkt und - je nach Ausrichtung der seitlichen Öffnung der Epiduralkanüle - nach oben oder unten parallel zur Dura vorgeschoben. Da der Epiduralkatheter nicht auf das Perforationsloch in der Dura stoßen kann, wird die Gefahr einer versehentlichen intrathekalen Katheterlage so gut wie ausgeschlossen. Die Handhabung des dreiteiligen Bestecks ist für den Anwender einfach, weil die Spitze der Spinalkanüle zwangläufig in den Spinalkanal geradlinig eintritt und der Epiduralkatheter zwangläufig von dem Spinalkanal abgelenkt und in den Epiduralraum eingeführt wird. Ohne besondere Kunstfertigkeit werden die Spinalanästhesie und die Katheter-Epiduralanästhesie kombiniert durchgeführt, so daß der schnelle Wirkungseintritt der Spinalanästhesie und die Ausweitung des Anästhesiebereiches durch die Epiduralanalgesie mit beliebiger Analgesiedauer durch Nachspritzen voll ausgenutzt werden.

Zum leichten verklemmungsfreien Durchlaß der Spinalkanüle ist dar Loch in der Spitze der Epiduralkanüle oval und seine Längsachse verläuft parallel zur Längsachse der Epiduralkanüle. Das Loch kann durch Laserstrahl in die Stahlwandung der Epiduralkanüle gebrannt werden.

Auf der Spinalkanüle ist vorteilhafterweise mit Abstand zu ihrer Spitze ein koaxialer Führungsring befestigt, dessen Außendurchmesser geringfügig kleiner als der Innendurchmesser der Epiduralkanüle ist. Der koaxiale Führungsring, der aus Metall oder Kunststoff bestehen kann, dient dazu, die Spitze der überlangen Spinalkanüle in der Mitte der Epiduralkanüle zu zentrieren, so daß sie gezielt aus dem koaxialen Loch der Epiduralkanüle austritt. Eine solche Führung ist vorteilhaft, weil die Spinalkanüle außerordentlich dünn und instabil ist, so daß ihre Spitze beim Vorschub durch die Epiduralkanüle mit größerem Innendurchmesser zum Ausweichen und Verfehlen des axialen Loches neigt. Der Führungsring kann als umfangsmäßig geschlossenes kreiszylindrisches Rohr ausgebildet sein, das die Spinalkanüle vom Kanülenansatz bis in ihren Spitzenbereich umgibt. Das über die Epiduralkanüle vorstehende Ende der Spinalkanüle ist von dem Führungsring frei. Anstatt eines langen Rohres kann ein kurzer Rohrteil als Führungsring verwendet werden, der sich nur im spitzennahen Bereich der Spinalkanüle befindet.

In der Zeichnung ist ein Ausführungsbeispiel der Erfindung schematisch dargestellt. Es zeigt:
Fig. 1 eine Darstellung der Komponenten des Anästhesiebesteckes,
Fig. 2 bis 6 die verschiedenen Stadien der Anwendung des Kathetersets, und
Fig. 3A den spitzennahen Teil des Anästhesiebestecks im Zustand der Figuren 3 und 4 in vergrößertem Maßstab.

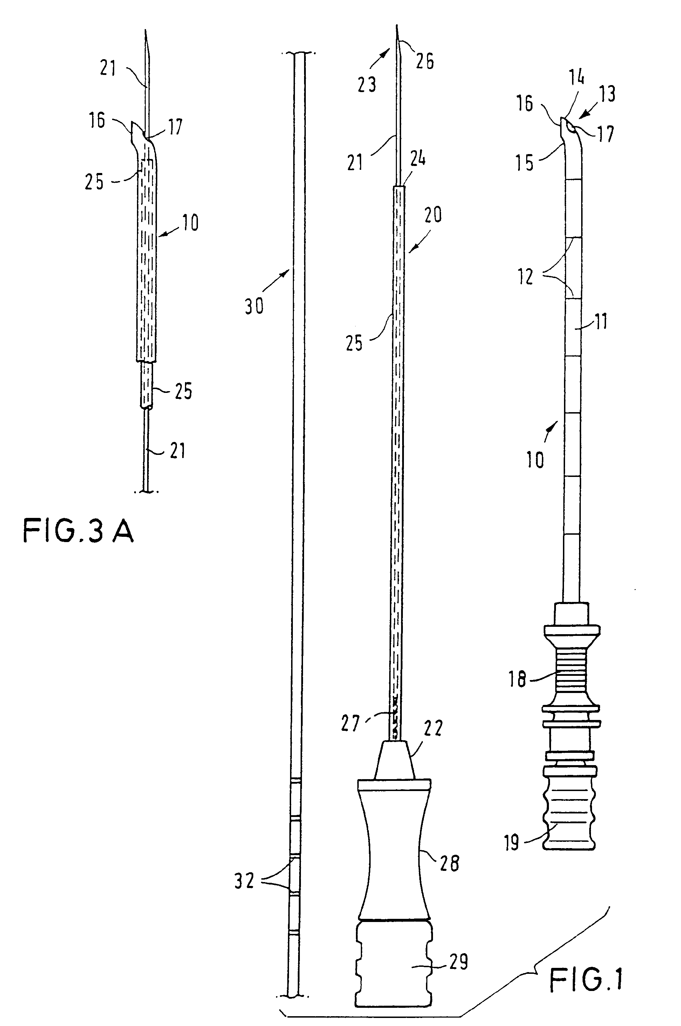

Gemäß Figur 1 ist eine Epiduralkanüle 10 vorgesehen, die aus einem geraden Kanülenrohr 11 aus Stahl mit Markierungsringen 12 zur Kontrolle der Einschublänge besteht. Die Spitze 13 der Epiduralkanüle 10 ist nach einer Seite abgebogen, so daß eine Krümmung mit einer äußeren Wand 14 und einer inneren Wand 15 entsteht und eine endständige Öffnung 16 seitwärts gerichtet ist. Die Öffnung 16 verläuft im wesentlichen parallel zur zylindrischen Wand des Kanülenrohres 11 und ist mit einem für die Gewebepunktion geeigneten Schliff versehen. Es handelt sich bei der Epiduralkanüle 10 um eine Kanüle des Tuohy-Typs, in der ein Mandrin, vorzugsweise aus Kunststoff, steckt, der durch sein Griffstück 19 symbolisiert ist, das in einem Kanülenansatz 18 des Kanülenrohres 11 steckt. Der Mandrin besitzt an seiner Spitze eine Abschrägung, die bei vorgegebener Ausrichtung des Mandrins in dem Kanülenrohr 11 mit der Öffnung 16 bündig abschließt. In der Außenwand 14 der Krümmung der Spitze 13 des Kanülenrohres 11 ist ein ovales Loch 17 ausgebildet. Die Längsachse des ovalen Loches 17 verläuft parallel zur Längsachse des Kanülenrohres 11 und das Zentrum des Loches 17 ist auf der Längsmittelachse des Kanülenrohres 11 zentriert.

Die Spinalkanüle 20 ist länger und sehr viel feiner als die Epiduralkanüle 10. Sie besteht aus einem dünnen Kanülenrohr 21, dessen Spitze 23 eine schräggerichtete Öffnung 26 mit einem scharfen Anschliff aufweist, der zur Erzielung minimalen Punktionstraumas als Quincke-Schliff ausgeführt sein kann. An dem spitzenfernen Ende des Kanülenrohres 21 ist ein Kanülenansatz 28 befestigt, der an dem kanülenseitigen Ende einen Außenkonus 22 und am anderen Ende ein inneres Anschlußelement zum Ansetzen einer Spritze aufweist. Der Außenkonus 22 dient zur Steckverbindung mit einem Innenkonus des Kanülenansatzes 18 der Epiduralkanüle 10. Das Lumen der Spinalkanüle 20 wird durch einen haarfeinen Metallmandrin 27 ausgefüllt, der mittels eines Griffstückes 29 aus der Spinalkanüle 20 herausziehbar ist. Das Kanülenrohr 21 ist über den größten Teil seiner Länge von einem kreiszylindrischen Kunststoffrohr 25 umgeben. Das Kunststoffrohr 25 ist an Dem Außenkonus 22 des Kanülenansatzes 28 befestigt und umspannt das Kanülenrohr 21 durch Aufschrumpfung fest, so daß es in dem Kunststoffrohr 25 stabilisiert ist. Der gleichmäßige Außendurchmesser des Kunststoffrohres 25 ist etwas kleiner als der Innendurchmesser des Kanülenrohres 11 der Epiduralkanüle 10 und die Länge des Kunststoffrohres 25 ist der Länge des Kanülenrohres 11 bis zum Beginn der Krümmung angepaßt. Das über den geraden stumpfen Rand 24 des Kunststoffrohres 25 vorstehende Stück der Spinalkanüle ragt aus den Loch 17 der Epiduralkanüle 10 heraus, wenn die Spinalkanüle 20 durch die Epiduralkanüle 10 geschoben und ihre Spitze 23 infolge der Führung durch das Kunststoffrohr 25 zentriert durch das Loch 17 der Epiduralkanüle 10 hindurchgetreten ist.

Zu dem Anästhesiebesteck gehört außerdem ein Epiduralkatheter 30. Dieser besteht aus einem langgestreckten flexiblen Katheterschlauch, dessen Außendurchmesser etwa dem Außendurchmesser des Kunststoffrohres 25 auf der Spinalkanüle 20 entspricht, d.h. etwas kleiner als der Innendurchmesser der Epiduralkanüle 10 ist. An der Spitze des Epiduralkatheters 30 befindet sich eine endständige koaxiale Öffnung mit gerundeten Kanten. Am entgegengesetzten Ende ist ein nicht gezeichneter Konnektor zum Anschluß einer Spritze oder dergleichen befestigt. Ferner ist der Katheterschlauch am rückwärtigen Ende mit Markierungen 32 zur Kontrolle der Einschublänge versehen.

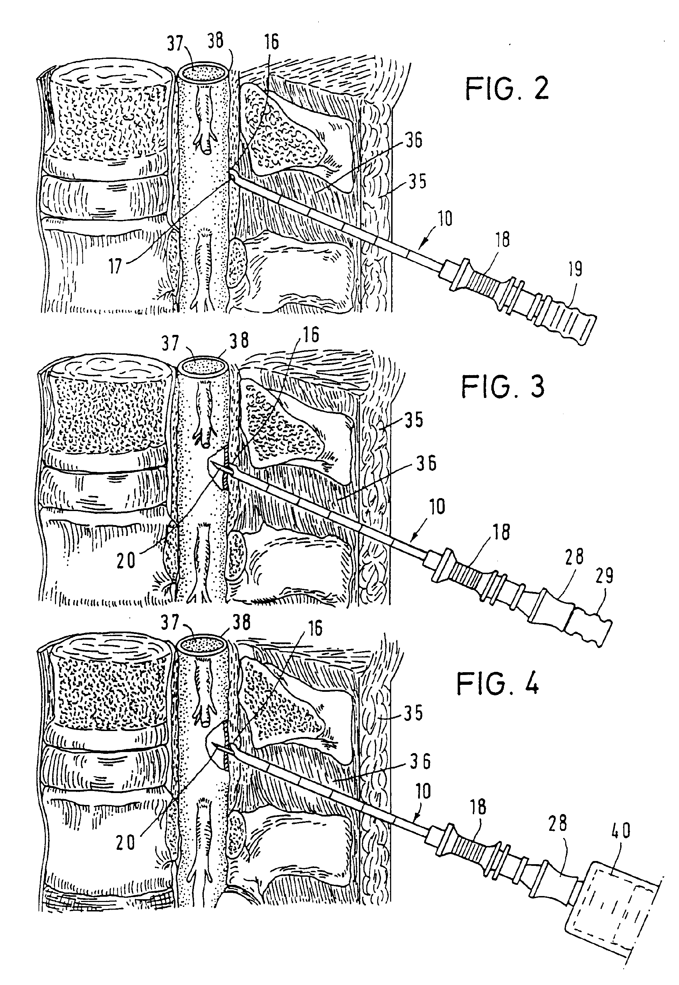

Die Anwendung des Anästhesiebesteckes gemäß Figur 1 ist in Den Figuren 2 bis 6 veranschaulicht.

Zunächst werden mit der von dem Mandrin ausgefüllten Epiduralkanüle 10 die Haut 35 und Muskelgewebe 36 durchstochen, bis die äußere Wand 14 der Spitze 13 der Epiduralkanüle 10 an der Außenseite der den Spinalkanal 37 umschließenden Dura 38 anliegt. Die Öffnung 16 der Epiduralkanüle 10 ist dabei vorteilhaft nach oben gerichtet und das Loch 17 weist gegen die Dura 38 (Fig. 2).

Nach Herausziehen des Mandrins aus der Epiduralkanüle 10 wird die Spinalkanüle 20 mit dem in ihr befindlichen Mandrin 27 in die Epiduralkanüle 10 bis nach intrathekal eingeschoben. Hierbei wird sie von dem Kunststoffrohr 25 in der Mitte der Epiduralkanüle 10 zentriert, so daß ihre Spitze 26 durch das Loch 17 austritt und die Dura 36 perforiert (Fig. 3).

Wenn Die Spitze 26 der Spinalkanüle 20 den Spinalkanal 37 erreicht hat, wird der Mandrin 27 aus der Spinalkanüle 20 herausgezogen und es wird mit dem Anschlußelement des Kanülenansatzes 28 ein passender Ansatz einer Spritze 40 gekoppelt, aus der Analgetikum für die Spinalanästhesie in den Spinalkanal 37 injiziert wird (Fig. 4).

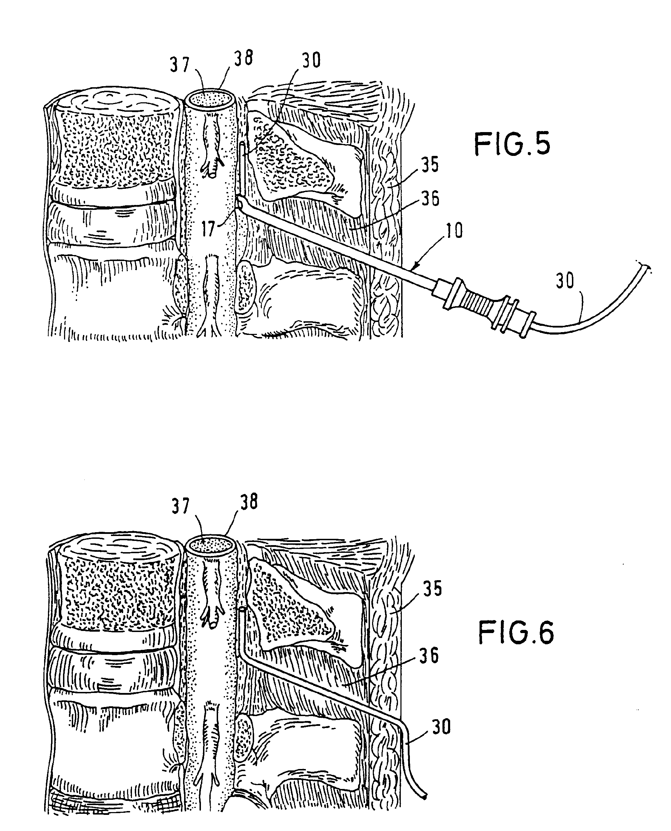

Nachdem die Spinalkanüle 20 aus der Epiduralkanüle 10 herausgezogen worden ist, steht die Epiduralkanüle 10 als Einführhilfe für den Epiduralkatheter 30 zur Verfügung (Fig. 5). Der Epiduralkatheter 30 wird durch den Kanülenansatz 18 und das Kanülenrohr 11 hindurch nach vorne geschoben, bis er gegen die Krümmung an der Spitze der Epiduralkanüle 10 trifft. Da er nicht durch das axiale Loch 17 paßt, wird er der Kanülenbiegung folgend zur Seite abgelenkt und gleitet entlang der Dura 38 nach oben. Ein Eindringen des Epiduralkatheters 30 in den Spinalraum 37 wird mit Sicherheit vermieden und die Gefahr einer versehentlichen intrathekalen Katheterlage ist nahezu ausgeschlossen. Anschließend wird die Epiduralkanüle 10 von dem Epiduralkatheter 30 axial abgezogen, so daß sich die in Figur 6 gezeigte Situation ergibt. Schließlich wird an das rückwärtige Katheterende ein nicht dargestellter Konnektor angeschlossen, um durch eine angesetzte Spritze Analgetikum in den Epiduralraum vor der Dura 38 einzuspritzen. Beliebige Analgesiedauer durch Nachspritzen ist auf diese Weise erreichbar und auch für postoperative Analgesie ist die Epiduralanästhesie gut geeignet.

## Patentansprüche

1. Anästhesiebesteck, bestehend aus einer geraden Epiduralkanüle (10) mit abgebogener Spitze (13), die eine seitwärts gerichtete angeschliffene Öffnung (16) aufweist und in der äußeren Wand (14) der Krümmung der Spitze (13) mit einem auf der Längsmittelachse der Epiduralkanüle (10) zentrierten Loch (17) versehen ist,
einer Spinalkanüle (20), die länger und dünner als die Epiduralkanüle (10) und durch diese so hindurchschiebbar ist, daß sie mit ihrem vorderen Ende aus dem Loch (17) der Epiduralkanüle (10) vorsteht,
und einem durch die Epiduralkanüle (10) schiebbaren Katheter (30),
**dadurch gekennzeichnet,** daß auf der Spinalkanüle (20) mit Abstand zu ihrer Spitze (23) ein koaxialer Führungsring befestigt ist, dessen Außendurchmesser geringfügig kleiner als der Innendurchmesser der Epiduralkanüle (10) ist,
und daß das Loch (17) kleiner als der Außendurchmesser des Epiduralkatheters (30) ist.

2. Anästhesiebesteck nach Anspruch 1,
**dadurch gekennzeichnet**, daß der Führungsring als umfangsmäßig geschlossenes kreiszylindrisches Rohr (25) ausgebildet ist.

3. Anästhesiebesteck nach Anspruch 2,
**dadurch gekennzeichnet**, daß das Rohr (25) aus einem Kunststoffschlauch besteht, der sich unter Freilassung eines spitzennahen Kanülenabschnittes über die gesamte Länge der Spinalkanüle (20) erstreckt.

4. Anästhesiebesteck nach einem der Ansprüche 1 bis 3,
**dadurch gekennzeichnet**, daß das Loch (17) oval ist und daß seine Längsachse parallel zur Längsachse der Epiduralkanüle (10) verläuft.

## Claims

1. An anaesthesia set comprising a straight epidural cannula (10) with a bent tip (13) having a laterally directed ground opening (16) and being provided with a hole (17) in the exterior wall (14) of the bend of said tip (13), said hole being centered on the longitudinal centre axis of said epidural cannula (10),
a spinal cannula (20) being longer and thinner than said epidural cannula (10) and being adapted to be advanced through the same such that its front end projects from said hole (17) of said epidural cannula (10),
and a catheter (30) adapted to be advanced through said epidural cannula (10),
characterized in that
a coaxial guide ring is mounted on said spinal cannula (20) at a distance from the tip (23) thereof, the outer diameter of said ring being slightly smaller than the inner diameter of said epidural cannula (10)
and said hole (17) being smaller than the outer diameter of the epidural catheter (30).

2. The anaesthesia set of claim 1, wherein said guide ring is shaped as a circumferentially closed circularly cylindrical tube (25).

3. The anaesthesia set of claim 2, wherein said tube (25) consists of a hose of plastics material that extends over the entire length of said spinal cannula (20), leaving a cannula section close to the tip uncovered.

4. The anaesthesia set of one of claims 1 to 3, wherein said hole (17) is oval in shape, and the longitudinal axis thereof extends parallel to the longitudinal axis of said epidural cannula (10).

## Revendications

1. Trousse pour l'anesthésie, composée d'une canule épidurale (10) rectiligne à pointe (13) recourbée, présentant une ouverture (16) à surface rectifiée orientée latéralement et pourvue, dans la paroi extérieure (14) de la courbure de la pointe (13), d'un trou (17) centré sur l'axe médian longitudinal de la canule épidurale (10),
d'une canule spinale (20), plus longue et plus mince que la canule épidurale (10) et susceptible d'être enfilée à travers celle-ci, de telle façon qu'elle fasse saillie, par son extrémité avant, hors du trou (17) de la canule épidurale (10),
et d'un cathéter (30), susceptible d'être enfilé à travers la canule épidurale (10),
caractérisée en ce que, sur la canule spinale (20), à distance de sa pointe (23), est fixée une bague de guidage coaxiale, dont le diamètre extérieur est légèrement inférieur au diamètre intérieur de la canule épidurale (10),
et en ce que le trou (17) est d'un diamètre inférieur au diamètre extérieur de la canule épidurale (30).

2. Trousse pour l'anesthésie selon la revendication 1, caractérisée en ce que la bague de guidage est réalisée sous forme de tube cylindrique circulaire (25), à périphérie fermée.

3. Trousse pour l'anesthésie selon la revendication 2, caractérisée en ce que le tube (25) est composé d'un tuyau en matière synthétique, s'étendant sur toute la longueur de la canule spinale (20), tout en laissant dégagée une section de canule proche de la pointe.

4. Trousse pour l'anesthésie selon l'une des revendications 1 à 3, caractérisée en ce que le trou (17) est ovale et en ce que son axe longitudinal s'étend parallèlement à l'axe longitudinal de la canule épidurale (10).
